**Europäisches Patentamt**

**European Patent Office**

**Office européen des brevets**

(19)

(11) Veröffentlichungsnummer: **0 033 567**
**A2**

(12) # EUROPÄISCHE PATENTANMELDUNG

(21) Anmeldenummer: **81200086.7**

(51) Int. Cl.³: **A 61 B 5/02**

(22) Anmeldetag: **23.01.81**

(30) Priorität: **04.02.80 DE 3004011**

(43) Veröffentlichungstag der Anmeldung:
**12.08.81 Patentblatt 81/32**

(84) Benannte Vertragsstaaten:
**CH DE FR GB IT LI NL SE**

(71) Anmelder: **Philips Patentverwaltung GmbH**
**Steindamm 94**
**D-2000 Hamburg 1(DE)**

(84) Benannte Vertragsstaaten:
**DE**

(71) Anmelder: **N.V. Philips' Gloeilampenfabrieken**
**Pieter Zeemanstraat 6**
**NL-5621 CT Eindhoven(NL)**

(84) Benannte Vertragsstaaten:
**CH FR GB IT LI NL SE**

(72) Erfinder: **Thees, Richard Dr. rer.nat.**
**Piusstrasse 17**
**D-5100 Aachen(DE)**

(72) Erfinder: **Wilden, Rolf**
**Brandstrasse 56**
**D-5106 Roetgen(DE)**

(74) Vertreter: **Hartmann, Heinrich et al,**
**Philips Patentverwaltung GmbH Steindamm 94**
**D-2000 Hamburg 1(DE)**

(54) **Blutdruck-Messvorrichtung.**

(57) Die Erfindung betrifft eine Blutdruck-Meßvorrichtung mit einer auf besonders einfache Weise selbsttätig aufpumpbaren Manschette. Das Aufpumpen erfolgt durch Heizung einer Flüssigkeit, die in einem mit der Manschette gekoppelten Reservoir enthalten ist. Dadurch übersteigt der Dampfdruck in der Manschette den Atmosphärendruck, so daß sich die Manschette aufbläht. Der Dampfdruck der Flüssigkeit muß bei Zimmertemperatur niedriger sein als der Atmosphärendruck und bei einer darüberliegenden, vom Menschen wenigstens kurzzeitig noch ertragbaren Temperatur (z.B. 50° C) muß der Dampfdruck oberhalb des Atmosphärendrucks liegen. Der Druck in der Manschette kann in üblicher Weise durch einen geeigneten Druckaufnehmer gemessen werden. Es kann jedoch auch die Temperatur gemessen und daraus auf den Druck geschlossen werden, weil zwischen Temperatur und Dampfdruck in der Manschette ein eindeutiger Zusammenhang besteht.

FIG.1

Croydon Printing Company Ltd.

Blutdruck-Meßvorrichtung

Die Erfindung betrifft eine Blutdruck-Meßvorrichtung mit einer an einen Körperteil, vorzugsweise einem Finger, anlegbaren Manschette und mit einer Meßeinrichtung zur Messung des Druckes in der Manschette. Zur Blutdruck-Messung wird dabei die Manschette aufgepumpt oder aufgeblasen, bis der Blutfluß durch die Arterie unterbunden ist. Wird der Druck in der Manschette allmählich abgesenkt, wird bei einem bestimmten Druck (systolischer Blutdruck) wieder der Puls in der Arterie fühlbar bzw. es tritt an der Arterie ein mit dem Puls synchroner Ton ("Korotkoff-Geräusch") auf. Wird der Druck in der Manschette weiter erniedrigt, wird bei einem bestimmten Druck (diastolischer Blutdruck) das Korotkoff-Geräusch plötzlich leiser. Bei der Blutdruck-Messung geht es vor allem um die Erfassung dieser beiden Blutdruckwerte.

Bei den bekannten Geräten dieser Art wird die Manschette im allgemeinen an den Oberarm des Patienten angelegt. Es gibt jedoch auch Blutdruck-Meßvorrichtungen, bei denen die Manschette über den Finger geschoben wird (vgl. z.B. DE-OS 18 17 089 und 28 42 337). Damit lassen sich einwandfreie Blutdruck-Messungen durchführen, wenn darauf geachtet wird, daß der Finger, an den die Manschette angelegt wird, bei der Messung sich etwa in Höhe des Herzens befindet, so daß statische Druckdifferenzen keine Rolle spielen.

Das Aufpumpen bzw. Aufblasen der Manschette erfolgt bei einigen Blutdruck-Messungen von Hand, was eine gewisse Erfahrung voraussetzt, damit der Druck in der Manschette nicht zu hoch oder zu niedrig ausfällt. Bei anderen Blutdruck-Meßvorrichtungen muß der Benutzer lediglich eine Pumpe einschalten, die die Manschette aufpumpt. Solche Bludruck-Meßvorrichtungen sind aber teuer.

Es ist Aufgabe der vorliegenden Erfindung, eine Blutdruck-Meßvorrichtung zu schaffen, die mit einfachen Mitteln das nicht-manuelle Aufpumpen bzw. Aufblasen der Manschette erlaubt.

Diese Aufgabe wird erfindungsgemäß dadurch gelöst, daß ein Teil der Manschette bzw. ein mit der Manschette in Verbindung stehendes Reservoir mit einer Flüssigkeit gefüllt ist, deren Siedepunkt zwischen 290 K und 340 K liegt, und daß in der Flüssigkeit ein an eine Strom- oder Spannungsquelle anschließbares Heizelement angeordnet ist.

Das Aufpumpen erfolgt dabei also dadurch, daß das Heizelement an die Strom- bzw. Spannungsquelle angeschlossen wird. Dadurch steigt die Temperatur in der Flüssigkeit und damit deren Dampfdruck, bis der Atmosphärendruck überschritten wird und die Manschette aufgebläht wird.

Außer der angegebenen Temperaturabhängigkeit des Dampfdruckes sollte die Flüssigkeit eine möglichst niedrige Wärmekapazität und eine möglichst niedrige Verdampfungswärme aufweisen, damit die Energiezufuhr zum Aufblasen bzw. Aufblähen der Manschette klein gehalten werden kann. Geeignete Flüssigkeiten sind beispielsweise Diäthyläther oder Aceton.

Das Heizelement muß stets in die Flüssigkeit eintauchen. Wenn also die Flüssigkeit sich nicht in einem gesonderten Reservoir - zweckmäßigerweise unterhalb der Manschette - befindet, sondern in einem Teil der Manschette, muß dafür Sorge getragen werden, daß das Heizelement sich stets an der tiefsten Stelle der Manschette befindet, wo die Flüssigkeit zusammenläuft.

0033567

PHD 80-011 EP

Grundsätzlich kann die Erfindung auch bei Blutdruck-Meßgeräten mit an den Oberarm anzulegenden Manschetten eingesetzt werden. In diesem Fall ist jedoch das Manschettenvolumen größer, was ein größeres Flüssigkeitsvolumen erfordert, und dies setzt wiederum voraus, daß die Heizleistung für das Heizelement entsprechend höher ist.

Eine vorteilhafte Weiterbildung sieht vor, daß das Heizelement ein PTC-Widerstand ist. Aufgrund des positiven Temperaturkoeffizienten kann dabei eine bestimmte, von der Bemessung des PTC-Widerstandes und der Heizspannung abhängige Temperatur praktisch nicht überschritten werden, wenn die Heizspannungsquelle genügend niederohmig ist: Wenn die Temperatur steigen würde, würde der Widerstand des PTC-Widerstandes zunehmen, was eine Verringerung der in ihm erzeugten elektrischen Leistung zur Folge hätte, was ein Abnehmen der Temperatur bedeuten würde. Wenn hingegen die Temperatur abnehmen würde, würde auch der Widerstandswert abnehmen, die in dem PTC-Widerstand erzeugte Leistung würde steigen, so daß die Temperatur auch wieder steigen würde.

Eine Weiterbildung der Erfindung sieht vor, daß die Meßeinrichtung zur Messung des Druckes in der Manschette einen Temperaturfühler enthält sowie einen Speicher, in dem die Temperatur-Dampfdruckkennlinie der Flüssigkeit gespeichert ist, und eine Steuereinrichtung, die für die genannte Temperatur den zugehörigen Dampfdruck bestimmt. Anstelle der Druckmessung tritt hier also eine Temperatur-messung, wobei der der jeweils gemessenen Temperatur zugeordnete Druck aus der Temperatur-Dampfdruckkennlinie ermittelt wird.

Besonders einfach ist diese Temperatur-Messung nach einer Weiterbildung der Erfindung, wenn als Temperaturfühler der PTC-Widerstand dient. Der PTC-Widerstand wirkt also vor der Messung als Heizelement, während der Messung als Meßelement. Der ihn durchfließende Strom muß dabei während der Messung selbstverständlich wesentlich kleiner sein.

Eine andere Weiterbildung der Erfindung sieht vor, daß zur Druckmessung ein piezo-resistives Element in der Manschette bzw. dem Reservoir vorgesehen ist. Dieses piezo-resistive Element verändert seinen Widerstand mit dem Druck. Die durch die Korotkoff-Geräusche hervorgerufenen Druckänderungen machen sich bei einem solchen Element als Widerstandsänderungen bemerkbar, und diese Widerstandsänderungen können zur Bestimmung des systolischen bzw. diastolischen Blutdrucks herangezogen werden.

Die Erfindung wird nachstehend anhand eines in der Zeichnung dargestellten Ausführungsbeispiels näher erläutert. Es zeigen

Fig. 1 einen Schnitt durch ein Gehäuse einer Blutdruck-Meßvorrichtung,

Fig. 2 eine Draufsicht auf eine solche Vorrichtung,

Fig. 3 das Reservoir mit dem Heizelement und einem Wandlerelement,

Fig. 4 eine Schaltung, in der ein PTC-Widerstand einerseits als Heizelement und andererseits als Meßelement dient,

Fig. 5 eine Schaltung zum Erfassen von Korotkoff-Geräuschen und

Fig. 6 eine Steuerschaltung mit einem Mikroprozessor.

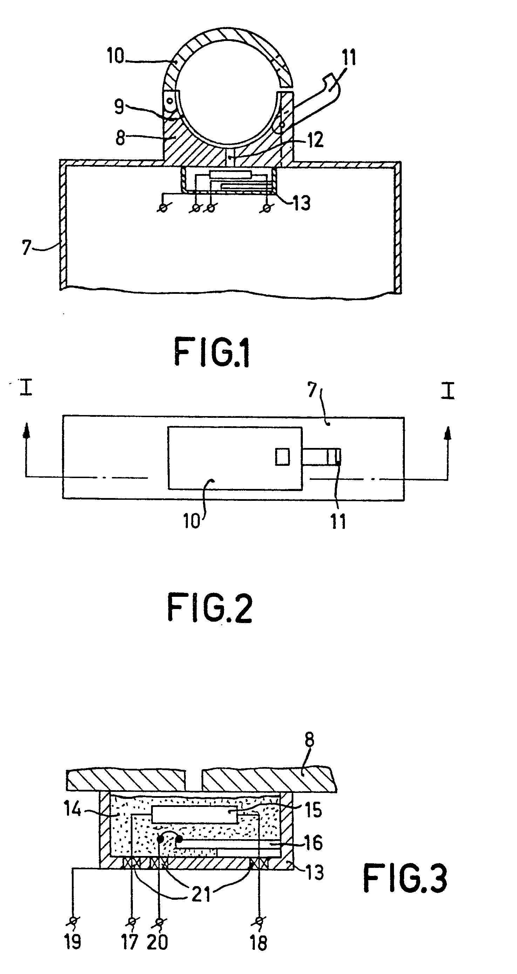

Wie aus den Fig. 1 und 2 ersichtlich, befindet sich auf einem Gehäuse 7 ein halbkreisförmiges Unterteil 8, in das

eine Manschette 9 eingelegt ist. Bei der Messung legt der Patient seinen Finger auf die Manschette, wonach der Finger mit Hilfe eines klappbaren Oberteils 10, das mit einem elastischen Haken 11 gesichert werden kann, fixiert wird. Wie insbesondere aus Fig. 3 hervorgeht, befindet sich unterhalb des halbkreisförmigen Unterteils ein Reservoir 13, das mit einer Flüssigkeit 14 gefüllt ist und das über eine Bohrung 12 mit der Manschette 9 in Verbindung steht.

Die Flüssigkeit in dem Reservoir muß folgende Eigenschaften haben:
- Bei Raumtemperatur (293 K) überschreitet der Dampfdruck nicht den Atmosphärendruck,
- bei einer für den Menschen kurzzeitig nicht als unangenehm empfundenen Temperatur (300 K bis 340 K) sollte der Dampfdruck ca. 200 mbar bis 280 mbar oberhalb des Atmosphärendruckes liegen,
- die Wärmekapazität und die Verdampfungswärme der Flüssigkeit sollten möglichst niedrig sein.

Geeignete Flüssigkeiten sind z.B. Diäthyläther und Aceton. Diäthyläther hat bei 20°C einen Dampfdruck von rund 550 mbar und bei 40°C einen Dampfdruck von rund 1200 mbar. Aceton hat bei Zimmertemperatur einen Dampfdruck von etwa 250 mbar und bei 60°C einen Dampfdruck von rund 1200 mbar. Weitere geeignete Flüssigkeiten sind fluorierte Kohlenwasserstoffe. Zwei dieser Flüssigkeiten sind $C\,Cl\,F_2 - C\,Cl_2\,F$ und $C\,Cl_3 - C\,F_3$, die unter der Bezeichnung Freon-113 im Handel sind.

Die Erwärmung der Flüssigkeit erfolgt mittels eines PTC- Widerstandes 15, der mit seinen über geeignete Durchführungen 21 aus dem Reservoir geführten Anschlüssen 17 und 18 an eine Spannungsquelle geeigneter Größe angeschlossen wird. PTC-Widerstände (Kaltleiter) haben den Vorteil, daß eine bestimmte Temperatur nicht überschritten werden kann, weil bei einem weiteren Ansteigen der Temperatur der Widerstandswert des PTC-Widerstandes zunimmt, so daß die von der Spannungsquelle gelieferte, in dem PTC-Widerstand in Wärme umgesetzte elektrische Leistung abnimmt, was einem Temperaturanstieg entgegenwirkt. Der PTC-Widerstand und die Spannungsquelle müssen so aufeinander abgestimmt gewählt sein, daß mit Sicherheit eine bestimmte Temperatur (z.B. 330 K) nicht überschritten wird.

Wenn der Dampfdruck in dem Reservoir den Atmosphärendruck überschreitet, tritt Dampf aus dem starren Reservoir 13 über die Bohrung 18 in die Manschette 9 und bläht diese auf, bis die durch den PTC-Widerstand vorgegebene Temperaturgrenze und damit ein bestimmter Druck, der höher sein muß als der im allgemeinen zu erwartende systolische Druck, erreicht ist. Die Manschette schnürt dann die Fingerarterie ab. Nach Abschalten der Spannung am PTC-Widerstand 15 sinkt die Temperatur der Flüssigkeit ab - und damit auch der Dampfdruck - und zwar in dem Maße wie Wärme über das umgebende Gehäuse abgegeben werden kann. Versuche haben gezeigt, daß es ausreicht, wenn das Unterteil 8 aus Metall (z.B. Messing) besteht. In diesem Fall ergibt sich ein Druckabfall von ca. 6 mbar/sec. Die während des Druckabfalls auftretenden Korotkoff-Geräusche werden mit Hilfe eines geeigneten, z.B. piezoelektrischen Wandlers 16, der ebenfalls in dem Reservoir angeordnet ist, in elektrische Signale umgewandelt und über die Anschlüsse 19 und 20 einer geeigneten Auswerteschaltung zugeleitet, die - ebenso wie die Spannungsquelle zur Heizung des PTC-Widerstandes - und die Schaltung zur Druck- bzw. Temperaturmessung sowie eine Steuerschaltung in dem Gehäuse 7 untergebracht ist.

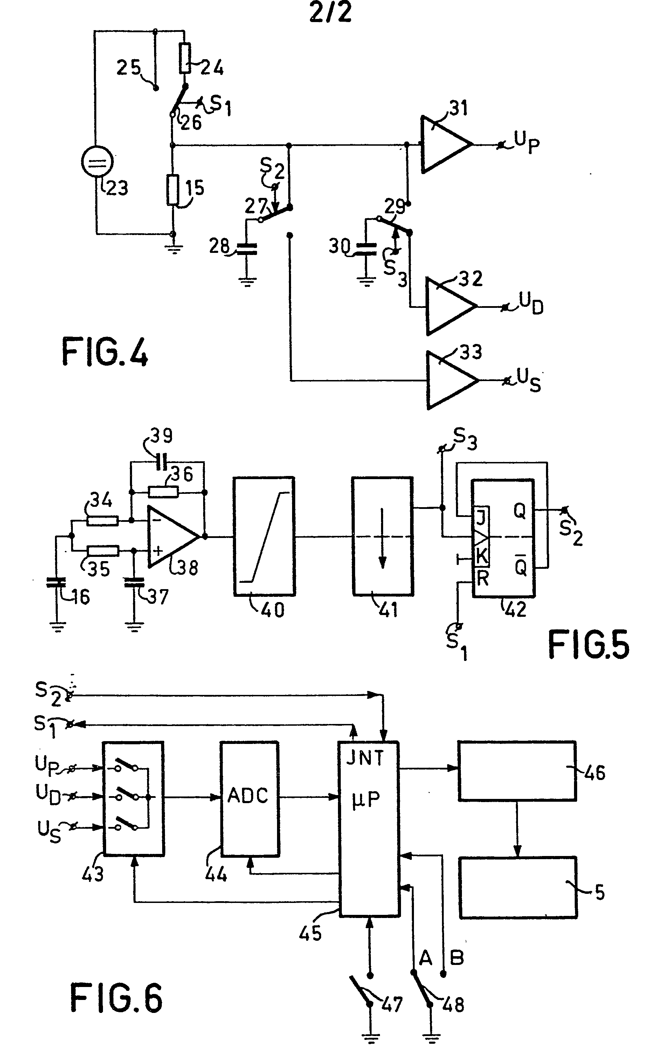

Die Schaltung zum Betreiben des PTC-Widerstandes 15 als Heiz- und Meßelement ist in Fig. 4 dargestellt. In der ersten Untersuchungsphase wird der PTC-Widerstand 15 über
den Kontakt 25 eines - vorzugsweise elektronisch - am
Steuereingang $S_1$ gesteuerten Umschalters 26 an eine Gleichspannungsquelle 23 angeschlossen. Der PTC-Widerstand 15
und mit ihm die in dem Reservoir 13 enthaltende Flüssigkeit wird dabei auf eine von der Bemessung der Spannungsquelle 23 und der Temperatur-Widerstandskennlinie des
PTC-Widerstandes 15 abhängige Grenztemperatur aufgeheizt.
Nach einer Zeitspanne, die so bemessen ist, daß auch im
ungünstigsten Fall die Flüssigkeit die Grenztemperatur
erreicht hat, wird der Umschalter 26 durch einen Impuls
am Steuereingang $S_1$ umgeschaltet in die in der Zeichnung
dargestellte Stellung, in der sie in Reihe mit einem Vorwiderstand 24 an die Spannungsquelle 23 angeschlossen ist.
Der Vorwiderstand 24 ist so bemessen, daß er auch bei der
Grenztemperatur groß im Vergleich zum Widerstandswert
des PTC-Widerstandes 15 ist, so daß der PTC-Widerstand
von einem im wesentlichen konstanten Meßstrom (der viel
kleiner ist als der Strom, der den PTC-Widerstand in der
Ausheizphase durchfließt) durchflossen wird. Die Spannung
am PTC-Widerstand ist dabei ein Maß für seinen Widerstandswert, der wiederum ein Maß für die jeweilige Temperatur
in der Flüssigkeit ist, die ihrerseits - jedenfalls bei
einem Dampfdruck oberhalb des Atmosphärendrucks - in eindeutiger Weise mit dem Druck in der Manschette zusammenhängt; d.h. die Spannung am PTC-Widerstand 15 ist ein
Maß für den Manschettendruck. Diese Spannung wird durch
einen Verstärker 31 verstärkt.

Anstatt das Heizelement während einer vorgebbaren Zeitspanne heizen zu lassen, ist es auch möglich, nur solange
zu heizen, bis eine vorgegebene Temperatur, also ein
vorgegebener Dampfdruck erreicht ist. Dazu muß während
des Heizens in kurzen Zeitintervallen die Temperatur
(Druck) gemessen werden, beispielsweise dadurch, daß der
Umschalter 26 kurzzeitig umgeschaltet wird. Die Messung
kann aber auch durch einen separaten Temperaturfühler, z.B. einen NTC-Widerstand erfolgen. Damit kann auch eine
Überhitzung des PTC-Widerstandes, die bei unsachgemäßer
Behandlung (unzulässige Wärmeisolation des Gehäuses)
dadurch eintreten könnte, daß die Temperatur, oberhalb der
der PTC-Widerstandswert mit steigender Temperatur wieder
abnimmt, überschritten wird, mit Sicherheit vermieden
werden.

Im einfachsten Fall könnte die Ausgangsspannung $U_p$ des Verstärkers 31 z.B. einem Drehspulinstrument zugeführt werden,
dessen Skala in Druckeinheiten geeicht ist, so daß diese
Skala gewissermaßen einen Speicher für den bei der benutzten
Flüssigkeit bei der jeweiligen Temperatur auftretenden
Druck darstellt.

Für die Diagnose ist nun nicht so sehr der Druck in der
Manschette, sondern vielmehr die Differenz zwischen dem
Manschettendruck und dem Atmosphärendruck von Bedeutung.
Da letzterer nicht immer konstant ist, können sich bei
der Auswertung Fehler ergeben. Falls diese Fehler nicht
toleriert werden können, können sie durch Subtraktion
des Atmosphärendruckes eliminiert werden. Dieser könnte
z.B. vor oder nach der Messung als derjenige Manschettendruck definiert werden, bei dem die Manschette gerade
aufgebläht wird bzw. wieder zusammenfällt. Bei dem erwähnten Beispiel mit einem Drehspulinstrument könnte
diese Korrektur dadurch erfolgen, daß bei dem betreffenden
Druck der Zeiger des Drehspulinstrumentes mechanisch auf
Null gestellt wird. Selbstverständlich ist aber auch eine
elektrische Subtraktion möglich.

Der systolische bzw. der diastolische Blutdruck wird mit
Hilfe eines weiteren Verstärkers 33 bzw. 32 sowie eines
Kondensators 28 bzw. 30 erfaßt, dessen eine Klemme an
Masse liegt und dessen andere Klemme über einen Umschalter
27 bzw. 29 wahlweise mit dem PTC-Widerstand 15, d.h. dem Eingang des Verstärkers 31, oder mit dem Eingang des
Verstärkers 33 bzw. 32 verbunden werden kann. Der Umschalter 27 bzw. 29 wird elektronisch an der Klemme $S_2$
bzw. $S_3$ gesteuert, an der Impulse aus der die Korotkoff-
Geräusche auswertenden Schaltung zugeführt werden. Wenn
der systolische Blutdruck erreicht ist, wird also durch
einen Impuls an der Klemme $S_2$ der Kondensatoranschluß mit
dem Eingang des Verstärkers 33 verbunden, dessen Eingang
genügend hochohmig ist, so daß der Kondensator sich nur
allmählich entladen kann.

Die Schaltung zur Auswertung der Korotkoff-Geräusche ist
in Fig. 5 dargestellt. Von dem piezoelektrischen Aufnehmer 16, der in dem Schaltbild durch eine Kapazität
repräsentiert wird, gelangen den Druckänderungen entsprechende elektrische Signale zu einem Verstärker 38, der als
Bandpaß für Signale im Frequenzbereich der Korotkoff-
Geräusche ausgelegt ist. Die untere Grenzfrequenz wird durch
das RC-Glied 35, 37 bestimmt und die obere Grenzfrequenz
durch das RC-Glied 36, 39. Über einen Schmitt-Trigger 40
und eine monostabile Kippstufe 41 wird beim Auftreten von
Korotkoff-Geräuschen bei jedem Herzpuls ein Impuls erzeugt,
der an der Klemme $S_3$ abgegriffen werden kann, die u.a. auch
den Steuereingang für den Umschalter 29 bildet. Dieser wird
also ständig hin und hergeschaltet, bis er in seiner gezeichneten Stellung stehenbleibt, wenn der Pegel der Korotkoff-Geräusche beim Erreichen des diastolischen Blutdruckwertes so gering geworden ist, daß der Schmitt-Trigger 40
nicht mehr anspricht.

An den Ausgang des Schmitt-Triggers 41 ist ein JK-Flipflop 42 angeschlossen, das lediglich beim Auftreten des
ersten Impulses an der Klemme $S_3$ an seinem Ausgang $S_2$, der
ua. auch den Umschalter 27 steuert, einen Impuls erzeugt,
und das durch einen Impuls an seiner mit dem Rückstelleingang R verbundenen Klemme $S_1$, deren Potential auch den Umschalter 26 steuert, zurückgesetzt werden kann.

In Fig. 6 ist eine Steuerschaltung zur Steuerung der in Fig. 4 und 5 dargestellten Schaltungen und zur Verarbeitung der von diesen Schaltungen gelieferten Meßwerte dargestellt. Zentrales Bauelement dieser Schaltung ist ein Mikroprozessor 45. Die dem Druck entsprechenden Ausgangsspannungen der Verstärker 31, 32 und 33 gelangen zunächst an einen vom Mikroprozessor gesteuerten Analogschalter 43, dem ein Analog-Digital-Wandler 44 nachgeschaltet ist, der sein analoges Eingangssignal in ein digitales Ausgangssignal umwandelt und dem Mikroprozessor zuführt. Der Mikroprozessor 45 steuert über einen Decoder 46 eine Anzeigeeinheit 5. Eine Starttaste 47 erzeugt ein Startsignal, das die Steuerung durch den Mikroprozessor 45 in Gang setzt, und ein Schalter 48 legt fest, welcher der beiden Blutdruckwerte (z.B. in Stellung A der systolische Blutdruck, in Stellung B der diastolische Blutdruck) ausgegeben wird.

Nach dem Betätigen der Starttaste werden alle Meßgrößenspeicher im Prozessor auf Null gesetzt. Gleichzeitig wird an die Klemme $S_1$ ein Signal ausgegeben, das den Umschalter 26 auf den Kontakt 25 umschaltet ("Heizen") und das Flipflop 42 zurücksetzt. Nach etwa 10 sec, d.h. einer Zeit, innerhalb der die Flüssigkeit auf eine genügend hohe Temperatur aufgeheizt ist, wird durch einen weiteren Impuls der Umschalter 26 in die in Fig. 4 dargestellte Stellung umgeschaltet. Der danach ablaufende Teil des Programmes kann jederzeit durch einen Impuls auf dem Interrupt-Eingang des Mikroprozessors unterbrochen werden, der mit der Klemme $S_2$ verbunden ist. Wie bereits erwähnt, wird dieser Impuls erzeugt, wenn die Differenz zwischen Manschettendruck und Atmosphärendruck dem systolischen Blutdruckwert entspricht. Die Spannung $U_{syst}$ am Ausgang des Verstärkers 33 ist daher bei dem gegebenen Atmosphärendruck ein Maß für den systolischen Blutdruck. Der Mikroprozessor 45 schaltet den Analogschalter 43 auf den Ausgang des Verstärkers 33, der über den Analog-Digital-Wandler 44 in ein digitales Signal umgewandelt und in einen Speicher des Mikroprozessors eingelesen wird. Aus einer in einem Festwertspeicher enthaltenen Tabelle, in der verschiedenen Spannungen verschiedene Drucke zugeordnet sind, wird der dem Meßwert $U_{syst}$ zugeordnete Druck interpoliert.

Anschließend wird der Analogschalter 43 mit dem Ausgang des Verstärkers 31 verbunden und die hier erzeugte Spannung $U_p$ wird mit einem Wert verglichen, der kleiner ist als der normalerweise zu erwartende diastolische Blutdruckwert. Solange der Druck noch nicht so weit abgesunken ist, wird der jeweilige Wert $U_p$ immer wieder eingelesen und verglichen. Dabei schalten die im Takte der Herzfrequenz an der Klemme $S_3$ erzeugten Impulse jedesmal den Umschalter 29 um, bis das Korotkoff-Geräusch verschwindet, wonach am Ausgang des Verstärkers 32 eine Spannung $U_{diast}$ anliegt, die dem diastolischen Druck entspricht. Wenn der Manschettendruck den vorerwähnten niedrigen Schwellwert unterschritten hat, endet dieser Programmteil, wonach der Analogschalter 43 mit dem Ausgang des Verstärkers 32 verbunden wird, dessen Ausgangsspannung in einen digitalen Wert gewandelt und in einen Speicher im Mikroprozessor eingelesen wird. Diesem Wert wird wiederum ein Druckwert zugeordnet. Nach Subtraktion eines dem – gegebenenfalls vorher gemessenen – Atmosphärendruck entsprechenden Wertes werden die beiden Werte angezeigt.

In dem in den Fig. 1 bis 3 dargestellten Ausführungsbeispiel wurde von einer halbkreisförmigen Fingermanschette ausgegangen, wie sie auch beispielsweise aus der DE-OS 28 42 337 bekannt ist. An ihrer Stelle kann aber auch eine den Finger bei der Messung vollständig umschließende Manschette verwendet werden, wie sie aus der DE-OS 18 17 089 bekannt ist.

Es ist nicht erforderlich, daß der Druck in der Manschette
über eine Temperaturmessung ermittelt wird. Er kann auch
direkt gemessen werden z.B. mittels eines piezo-resistiven
Elementes, das seinen Widerstand in Abhängigkeit von dem
darauf einwirkenden Druck ändert. Damit kann nicht nur der
quasi statische Druck in der Manschette, sondern auch die
durch die Korotkoff-Geräusche verursachten Druckschwankungen in der Manschette gemessen werden (die durch die Druckschwankungen verursachten Signale müssen in diesem Fall
durch einen geeigneten Hochpaß abgetrennt werden). Wenn
dabei die Flüssigkeit nicht geheizt wird, stellt sich in
der Manschette ein Druck ein, der dem jeweiligen Atmosphärendruck entspricht. Dieser kann mittels des piezo-resistiven
Elementes erfaßt und von den anschließend ermittelten
Werten $U_{syst}$ bzw. $U_{diast}$ subtrahiert werden.

PATENTANSPRÜCHE:

1.       Blutdruck-Meßvorrichtung mit einer an einen Körperteil, vorzugsweise einem Finger, anlegbaren Manschette und
mit einer Meßeinrichtung zur Messung des Druckes in der
Manschette, dadurch gekennzeichnet, daß ein Teil der
Manschette (9) bzw. ein mit der Manschette (9) in Verbindung stehendes Reservoir (13) mit einer Flüssigkeit
gefüllt ist, deren Siedepunkt zwischen 290 K und 340 K
liegt, und daß in der Flüssigkeit ein an eine Strom- oder
Spannungsquelle anschließbares Heizelement (15) angeordnet
ist.

2.       Blutdruck-Meßvorrichtung nach Anspruch 1, dadurch
gekennzeichnet, daß die Flüssigkeit Diäthyläther ist.

3.       Blutdruck-Meßvorrichtung nach Anspruch 1, dadurch
gekennzeichnet, daß die Flüssigkeit Aceton ist.

4.       Blutdruck-Meßvorrichtung nach Anspruch 1, dadurch
gekennzeichnet, daß die Flüssigkeit ein fluorierter
Kohlenwasserstoff ($C Cl F_2 - C Cl_2 F$ oder $C Cl_3 - C F_3$)
ist.

5.       Blutdruck-Meßvorrichtung nach einem der vorhergehenden Ansprüche, dadurch gekennzeichnet, daß das Heizelement (15) ein PTC-Widerstand ist.

6.       Blutdruck-Meßvorrichtung nach einem der vorhergehenden Ansprüche, dadurch gekennzeichnet, daß die Meßvorrichtung zur Messung des Druckes in der Manschette einen Temperaturfühler (15) enthält sowie einen Speicher, in dem die
Temperatur-Dampfdruckkennlinie der Flüssigkeit gespeichert
ist, und eine Steuereinrichtung (45), die für die genannte
Temperatur den zugehörigen Dampfdruck bestimmt.

7.    Blutdruck-Meßvorrichtung nach Anspruch 5 und 6,
dadurch gekennzeichnet, daß als Temperaturfühler der
PTC-Widerstand (15) dient.

8.    Blutdruck-Meßvorrichtung nach einem der Ansprüche 1
bis 5, dadurch gekennzeichnet, daß zur Druckmessung
ein  piezo-resistives Element in der Manschette bzw.
dem Reservoir vorgesehen ist.

FIG.1

FIG.2

FIG.3

1-Ⅱ-PHD 80-011

FIG.4

$U_P$

$U_D$

$U_S$

FIG.5

FIG.6